(19) Europäisches Patentamt
European Patent Office
Office européen des brevets

(11) Publication number: **0 467 433 A1**

(12) **EUROPEAN PATENT APPLICATION**

(21) Application number: **91201552.6**

(22) Date of filing: **19.06.91**

(51) Int. Cl.[5]: **A61K 37/40**, C07K 15/00, C07K 7/06

(30) Priority: **18.07.90 EP 90201951**

(43) Date of publication of application: **22.01.92 Bulletin 92/04**

(84) Designated Contracting States: **AT BE CH DE DK ES FR GB GR IT LI NL SE**

(71) Applicant: **AKZO N.V.**
**Velperweg 76**
**NL-6824 BM Arnhem(NL)**

(72) Inventor: **de Wied, David**
**Lassuslaan 41**
**NL-3723 LH Bilthoven(NL)**
Inventor: **van Nispen, Johannes Wilhelmus Franciscus Maria**
**Beethovengaarde 131**
**NL-5344 CE Oss(NL)**

(74) Representative: **Hermans, Franciscus G.M. et al**
**Patent Department AKZO N.V. Pharma Division P.O. Box 20**
**NL-5340 BH Oss(NL)**

(54) **Use of a centrally acting ATCH analog in the manufacture of a medicament.**

(57) Disclosed is the use of:

$H\text{-}MetO_2\text{-}Glu\text{-}His\text{-}Phe\text{-}D\text{-}Lys\text{-}Phe\text{-}X\text{-}(CH_2)_nCH_3$,

wherein X is NH or O, and n is a positive integer ranging from 7 to 17 for the manufacture of a medicament having selective centrally acting nerve regeneration activity, but essentially no peripheral nerve regeneration activity. Once manufactured, the medicaments may be used in the treatment of an animal suffering from central nerve degeneration or damage or may be used to determine the etiology of central versus peripheral nerve damage.

Fig 3

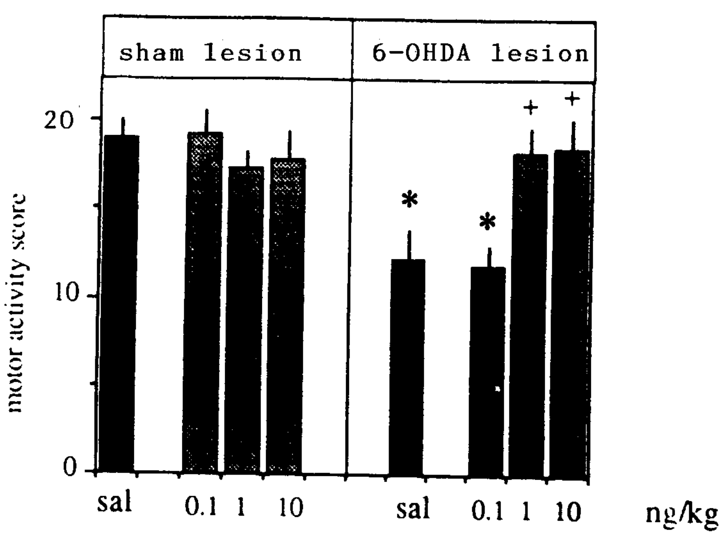


EP 0 467 433 A1

Background of the Invention

Field This invention relates to pharmaceutical compositions generally and to the use of an ACTH analog for the manufacture of a medicament having central nervous system activity specifically.

State of the Art: U.S. Patent No. 3,842,064 to Greven discloses psychopharmacologically active peptide derivatives of adrenocorticotropic hormone ("ACTH"). Similar peptides are described in U.S. Patent Nos. 4,104,371 and 4,110,322. Such peptide derivatives have been used to study the possibility of nerve regeneration.

U.S. Patent No. 4,550,099 discloses similar peptide derivatives such as:

H-$MetO_2$-Glu-His-Phe-D-Lys-Phe-OH

which can be used to stimulate the natural process of axonal regeneration. As used herein, "$MetO_2$" is methionylsulfone; "Glu" is glutamyl; "His" is histidyl; "Phe" is phenylalanyl; and "D-Lys" is D-lysyl. If no special optical configuration is stated, then the L-form is intended.

All of the disclosed peptide derivatives to date have had regenerative action both centrally (e.g. as determined by the bilateral injection of the neurotoxin 6-hydroxydopamine ("6-OHDA") and subsequent administration of the peptide derivative) and peripherally (as determined by "crush lesioning" of the peripheral nerve, and subsequent administration of the peptide derivative).

A peptide derivative having an aliphatic chain with a structure:

H-$MetO_2$-Glu-His-Phe-D-Lys-Phe-$OC_{10}H_{21}$

is disclosed in Van der Zee, CEEM, "Putative Neurotrophic Factors and Functional Recovery from Peripheral Nerve Damage in the Rat," Neurotrophic Peptides in Animal Models of Peripheral Nerve Regeneration and Diabetic Neuropathy, pp. 49-61 (16 Oct. 1989, The Hague, NL). It is reported that this peptide derivative is ineffective in facilitating the repair of damaged peripheral nerve, which would lead one to think that the peptide derivative is not effective in facilitating the repair of any damaged nerve. Id. at 57.

Summary of the Invention.

Surprisingly, it has been found that peptide derivatives of the formula:

H-$MetO_2$-Glu-His-Phe-D-Lys-Phe-$X(CH_2)_nCH_3$,

wherein X is NH or O, and n is a positive integer ranging from 7 to 17, has selective centrally acting nerve regeneration activity, but essentially no peripheral nerve regeneration activity.

These peptide derviatives have use in the manufacture of a medicament. Once manufactured, the medicaments may be used in the treatment of an animal suffering from central nerve degeneration or damage. For example, the medicaments are administered, on a regular basis, for example two to four times a day, to a animal, such as a human, believed to be suffering from a disease state susceptible to treatment by the compound. Such disease states include those wherein CNS function has degenerated either chemically or traumatically.

Brief Description of the Figures

FIG. 1 shows the effect of a preferred compound (wherein X is oxygen and n is 9) on passive avoidance behaviour. The peptides were given 1 hour prior to a 24 hour retention test. *: $P < 0.05$ vs. saline; Mann-Whitney U-test.

FIG. 2 depicts the influence of a preferred compound (wherein X is oxygen and n is 9) on motor activity of group housed rats tested under low light intensity. Groups of animals were s.c. injected with either saline or the compound (0.01-1.0 $\mu$g/kg) 1 hour before testing. Mean scores (± sem) of motor activity per group of animals are depicted. Results of one-way ANOVA: $F = 4.0$ (3,36), $P = 0.01$. *: Different from saline treatment ($P<0.05$; Newman Keuls).

FIG. 3 depicts the effect of subcutaneous treatment with a preferred compound (wherein X is oxygen and n is 9) on motor activity of rats with bilateral 6-OHDA lesions or sham lesions in the nucleus accumbens. The compound (0.1-10 ng/kg) was administered daily from day 1 to day 6 after the lesion. Motor activity of the rats was measured in a small open field 7 days after the lesion. Data represent mean motor activity scores (±sem). Results from two-way ANOVA: F(lesion) 20.1 (1,36) $P<0.001$; F(treatment) 3.3 (3,36) $P<0.03$; and F-(lesion x treatment) 9.6 (3,36) $P<0.01$. *: Different from sham lesioned animals ($P<0.05$; Newman Keuls); +: Different from 6-OHDA lesioned animals treated with saline ($P<0.05$; Newman Keuls).

FIG. 4 depicts the effect of oral treatment with a preferred compound (wherein X is oxygen and n is 9) on motor activity of rats with bilateral 6-OHDA lesions or sham lesions in the nucleus accumbens. The compound (1.0 or 10.0 $\mu$g/kg) was administered daily from day 1 to day 6 after the lesion. Motor activity of the rats was measured in a small open field 7 days after the lesion. Data represent mean motor activity scores (± sem). Results from two-way ANOVA: F(lesion) 70.6 (1,32) $P<0.001$; F-(treatment) 1.4 (2,32) n.s.; F(lesion x treatment) 5.6 (2,32) $P<0.01$. *: Different from lesioned animals

treated with saline ($P<0.01$)

Description of the Preferred Embodiments

Compounds for use in the medicament can be prepared as generally described in U.S. Patent No. 3,842,064 at column 3, line 5 through column 4, line 70, and column 7, line 1 through column 15, line 16, the contents of which are incorporated by this reference.

A process for making the compounds is thoroughly described in EXAMPLE I. By making appropriate substitutions of other alcohols for decanol in EXAMPLE I (e.g. in EXAMPLE I.A.1.(1.1)) other compounds can be synthesized. Other suitable alcohols for making the various compounds include: 2-decanol, 3,7-dimethyl-1-octanol, 1-undecanol, 2,4-diethyl-1-heptanol, 1-dodeca-nol, 2-dodecanol, 1-tridecanol, 1-tetradecanol, 2-tetra-decanol, 1-penta-decanol, 1-hexadecanol, 2-hexadecanol, 1-heptadecanol, oleyl alcohol, and 1-octadecanol.

Suitable amines include dibutylamine, di-sec-butylamine, diisobutylamine, N,N-diisopropylethylamine, N,N-dimethylhexylamine, N-methyl-dibutylamine, tertiaryoctylamine, diphenylamine, nonylamine, decylamine, N,N-dimethyloctylamine, 1,5-dimethylhexyl-2-ethylhexylamine, 1-methylheptyloctylamine, undecylamine, dihexylamine, dodecylamine, N,N-dimethyldodecylamine, 1-tetradecylamine, N-methyldioctylamine, and octadecylamine.

An especially preferred compound for use in the medicament has the formula:

H-$MetO_2$-Glu-His-Phe-D-Lys-Phe-$OC_{10}H_{21}$.

The compounds are useful in the manufacture of medicaments having use in treating symptoms of central nerve degeneration or disease. In the case of sudden damage to the central nervous system, the compounds are preferably administered to the patient as soon as possible after the damage occurs.

The compounds may also be useful in the differential diagnosis of central versus peripheral nerve damage, since they do not have appreciable peripheral action, but do have appreciable central nervous regeneration activities. In such a circumstance, the compound may be administered to an animal displaying symptoms of nervous degeneration of unknown location. If the symptoms improve, then it is known that the disease state must have at least some central nervous character. The compounds' central selectivity would be especially useful in analyzing a neurotoxicity associated with a chemical compound in an animal model.

As medicaments, the compounds are preferably administered subcutaneously, topically, intranasally, or locally (e.g. via an implant), although the preferred compound is active orally. When administered orally, the medicament containing the compound is preferably administered at least one-half hour before a meal.

The medicament for subcutaneous administration will generally be a dosage unit containing between 70 and 700 nanograms of the compound dissolved in saline. However, medicaments containing fewer nanograms of the compounds may also be used, if multiple subcutaneous injections are to be administered.

The medicament manufactured with the compounds may also be used as adjuvant therapy in the treatment of symptoms of central nervous degeneration. In such a case, the medicament is administered with other compounds useful in ameliorating such symptoms.

The dosage of compound administered is generally dependent on how the compound is to be administered. For example, doses of the compound:

H-$MetO_2$-Glu-His-Phe-D-Lys-Phe-$OC_{10}H_{21}$

as low as 0.5 to 5.0 nanograms/kilogram body mass (ng/kg), subcutaneously, facilitated passive avoidance behaviour in Wistar rats, while doses of 250 ng/kg attenuated the passive avoidance response. Doses of 0.1 to 1.0 microgams/kilogram ($\mu$g/kg) body mass of the same compound decreased motor activity of group housed rats tested under low light conditions. Furthermore subcutaneous (1.0 to 10.0 ng/kg) or oral (10 $\mu$g/kg) administration of the preferred compound accelerated functional recovery from 6-hydroxydopamine (6-OHDA)-induced lesions in the nucleus accumbens which cause motor hypoactivity. For topical administration (e.g. to a damaged eye in the form of drops, ointments, salves or creams) dosages such as used with the subcutaneous dosage form may be used.

The dosage forms used may be dosage forms for oral administration. Methods and compositions for making such dosage forms are well-known to those skilled in the art. For example, methods and compositions for making capsules, tablets and pills, containing active ingredients, are described in the standard reference, Chase et al., Remington's Pharmaceutical Sciences, (16th ed., Mack Publishing Co., Easton. PA, U.S.A., 1980) ("Remington's"), at pages 1553 through 1584. Methods of making powders, and their composition are described at pages 1535 through 1552 of the reference. Liquid dosage forms might also be used. Methods for making, and compositions of, solutions, emulsions, and suspensions are described at pages 1438

through 1462 of the text. Methods of coating pharmaceutical dosage forms, and making prolonged release pharmaceuticals are described at pages 1585-1613 of Remington's.

Oral pharmaceutical compositions containing sufficient quantities of the compound (e.g. 50 to 700 µg) may also contain other active compounds.

The compounds may also be used with implantable pharmaceutical devices such as those described in US Patent 4,767,628, the contents of which are incorporated by this reference. Then the device will contain sufficient amounts of compound (e.g. 150 to 2100 ng) to slowly release the compound (e.g. for more than a month).

Methods of making medicaments containing the compound for parenteral administration are described in Remington's at pages 1463 through 1497.

An especially preferred application of the compound is in the manufacture of ophthalmic preparations. Due to the compound's selective central nerve regenerating activity, the compound can be administered topically to a damaged eye where it is absorbed and acts locally. Methods for making ophthalmic preparations are described in Remington's at pages 1498 through 1517, the contents of which are incorporated by this reference.

The invention is further explained by reference to the following EXAMPLEs, which are presented to exemplify preferred embodiments of the invention and should not be construed as limitations thereof.

EXAMPLE I

A. Synthesis of H-MetO$_2$-Glu-His-Phe-D-Lys-Phe-OC$_{10}$H$_{21}$

1. Synthesis of Z-Phe-D-Lys(Boc)-Phe-OC$_{10}$H$_{21}$

1.1 Synthesis of H-Phe-OC$_{10}$H$_{21}$

6 Mmol of H-Phe-OH are suspended in 10 ml of decanol, the suspension cooled (-10 °C) and 6.66 ml of thionyl chloride are carefully added dropwise with stirring. After stirring overnight, the reaction flask was warmed for 4 hours at approximately 90-100 °C. The solvent was then evaporated under reduced pressure, the residue dissolved in 50 ml ethyl acetate and the solution washed with 5% aqueous NaHCO$_3$ solution and 10% NaCl solution (quantitative yield) Rf value in toluene-ethanol (4:1, V/V) is 0.46 on SiO$_2$.

1.2 Synthesis of Z-D-Lys(Boc)-Phe-OC$_{10}$H$_{21}$

6.12 Mmol Z-D-Lys(Boc)-ONp and H-Phe-OC$_{10}$H$_{21}$ (one equivalent) were dissolved in DMF (40 ml) and the solution kept overnight at room temperature. After evaporation, the residue was dissolved in ethyl acetate and the solution extracted with base and acid. The dipeptide was isolated after trituration with ether-petroleum ether (1:1, V/V) in 52% yield. M.p.: 95-97 °C; $[\alpha]_D^{19}$ = -4.0° (c = 1, DMF). Rf in toluene-ethanol (9-1, V/V) is 0.46 on SiO$_2$.

1.3 Synthesis of Z-Phe-D-Lys(Boc)-Phe-OC$_{10}$H$_{21}$

3.14 Mmol of Z-Phe-ONp were dissolved in DMF and H-D-Lys(Boc)-Phe-OC$_{10}$H$_{21}$ - [obtained by hydrogenation of Z-D-Lys(Boc)-Phe-OC$_{10}$H$_{21}$ in DMF with Pd/C as catalyst] was added. After standing overnight at room temperature, the solution was evaporated and the residue was dissolved in ethyl acetate. The peptide was precipitated by the addition of ether, yield 86.7% M.p.: 144-145 °C; $[\alpha]_D^{22}$ = -5 ° (c = 1, DMF); Rf in toluene-ethanol (4-1, V/V) is 0.50 on SiO$_2$.

2. Synthesis of Boc-MetO$_2$-Glu(OtBu)-His-Phe-D-Lys(Boc)-Phe-OC$_{10}$H$_{21}$

2.58 Mmol of Boc-MetO$_2$-Glu(OtBu)-His-N$_2$H$_3$ - (obtained as described in U.S. patent 3,842,064) were dissolved in 20 ml DMF, the solution cooled at -20 °C and 3 equivalent. of HCl in DMF were added followed by 2.84 mmol of isoamylnitrite. After 30 min. H-Phe-D-Lys(Boc)-Phe-OC$_{10}$H$_{21}$ [obtained by hydrogenation of the corresponding Z-peptide, 1.3, in DMF with Pd/C as catalyst]. After two days at 2-8 °C another 2.58 mmol of azide was added and the solution worked up 3 days later. Evaporation of the solvent, followed by dissolution in ethyl acetate and the usual acid-base extractions, the peptide was precipitated from DMF-H$_2$O (1-2, V/V). Yield: 91.7%; m.p.: 184-186 °C (decn.); $[\alpha]^{22}{}_D$ = -11.7 ° (c = 1, DMF). Rf in CHCl$_3$-MeOH-H$_2$O (7-3-0.5, by vol.) is 0.89; in 1-butanol-pyridine-acetic acid-water (16-3-1-4, by vol.) is 0.81 on SiO$_2$.

3. Synthesis of H-MetO$_2$-Glu-His-Phe-D-Lys-Phe-OC$_{10}$H$_{21}$

The protected hexapeptide, described Example I.A.2, was dissolved in TFA-H$_2$O(9:1, V/V, 2.29 mmol in 30 ml) and 0.76 ml anisole was added. After 30 min at room temperature the solution was added dropwise to ether. The precipitate was collected by filtration and washed with ether. After drying the solid was dissolved in tertiary butanol-water (1:1, V/V) and TFA ions exchanged for acetate ions, and the solution lyophilized. Purification was by chromatography on silica (solvent system 1-butanol-pyridine-acetic acid-water = 16-3-1-4, by vol.) Rf in 1-butanol-pyridine-acetic acid-water (8-3-1-4, by vol.) is 0.44 (SiO$_2$).

B. 6-OHDA Induced Motor Hypoactivity

A detailed description of the described lesion-

ing procedure is given in G. Wolterink et al, Brain Res., 507, pp. 92-100 (1990).

Male Wistar rats were used. The animals were housed in a room in groups of 5-6 animals with free access to food and tap water. At the moment of the operation the rats weighed approximately 140 grams. One hour prior to the operation the rats received an i.p. injection of desipramine (DMI, 25 mg/kg) to prevent destruction of noradrenergic nerve cells. The rats were anaesthetized with Hypnorm$^R$ (0.08 ml/100 grams bodyweight, i.m.) and secured in a stereotaxic apparatus. Stainless steel guide cannulae (outer diameter 0.6 mm) were bilaterally implanted into the rat brain, their tips ending in the nucleus accumbens (coordinates: 2.6 mm anterior to bregma, 2.7 mm lateral of the midline, 6.2 mm below the surface of the skull at the point of penetration, inserted at an angle of 12° to the midline, incisor bar at horizontal zero level).

The cannulae served to guide the needle of a Hamilton syringe (outer diameter 0.3 mm) through which 6-OHDA (8 µg/2 µl dissolved in saline containing 0.1% ascorbic acid) was injected over a 2 min period in the nucleus accumbens. Sham lesions were produced by injections of vehicle (saline containing 0.1% ascorbic acid). After the injection the guide cannulae together with the syringe was removed and the skin was stitched.

The animals received daily administrations of vehicle or different doses of the compound either s.c. (in 0.3 ml saline/rat) or orally (in 1.0 ml water/rat) from day 1 to day 6 after the lesion. On day 7 motor activity of the rats was assessed in the open field under bright light conditions (70 W light located 1.5 m above the testbox).

The following drugs were used: 6-hydroxydopamine (6-OHDA) Sigma Chem. Comp.). The compound ("Met0$_2$-Glu-His-Phe-D-Lys-Phe-O-$(CH_2)_9$-$CH_3$") was from Organon International B.V., Oss, The Netherlands. For s.c. administration the peptides were dissolved in saline using plastic containers on the day of use and the injection volume was 0.3 ml/rat. For oral administration the compound was dissolved in distilled water. The peptide solution was orally administered in a volume of 1 ml using an intra-gastric tube.

RESULTS

Passive avoidance behaviour

Treatment with the compound exerted a dual activity on passive avoidance behaviour (FIG. 1). Subcutaneous administration of the compound at doses of 0.5 ng/kg and 5 ng/kg 1 hour before the retention test facilitated avoidance behaviour while a dose of 250 ng/kg attenuated the avoidance response.

Environmentally induced changes in motor activity

Subcutaneous administration of the compound in graded doses to group housed rats tested under low light conditions resulted in a dose dependent decrease in motor activity which reached statistical significance at doses of 0.1 and 1.0 µg/kg (FIG. 2).

Lesion-induced motor hypoactivity

In rats with 6-OHDA-induced lesions in the nucleus accumbens that were treated with vehicle from day 1 to day 6 after the lesion motor activity was decreased at day 7 (FIG. 3). Subcutaneous treatment of 6-OHDA lesioned rats with the compound in doses of 1.0 and 10.0 ng/kg induced a complete recovery of motor activity on day 7 while a dose of 0.1 ng/kg was without effect. The compound, when given orally from day 1 to day 6 after the lesion at a dose of 10 µg/kg induced a significant recovery of motor activity on day 7 (FIG. 4). The dose of 1.0 µg/kg was not effective. Daily s.c. oral administration of the peptide did not affect motor activity of sham lesioned animals (FIGS. 3 and 4).

This example shows that the compound already facilitates the avoidance response at doses of 0.5 - 5.0 ng/kg. An inhibition of the avoidance response was induced by 250 ng/kg doses of the compound. The compound decreases motor activity of group housed rats tested under low light intensity at s.c. doses of 0.1 µg/kg. The compound facilitates functional recovery from impaired motor activity caused by 6-OHDA lesions in the nucleus accumbens at s.c. injected doses of 1 ng/kg. For oral administration of the compound the calculated $ED_{50}$ for the facilitation of functional recovery after a 6-OHDA lesion of the nucleus accumbens shows an effect after treatment with 10 µg/kg.

The compound accelerates functional recovery from 6-OHDA-induced impaired motor activity after oral administration to rats.

EXAMPLE II

In a similar manner as is described in EXAMPLE I, except substituting the appropriate alcohol for decanol in step 1.1, the following compounds are prepared:

a) H-Met0$_2$-Glu-His-Phe-D-Lys-Phe-O-$(CH_2)_7$-$CH_3$

b) H-Met0$_2$-Glu-His-Phe-D-Lys-Phe-O-$(CH_2)_8$-$CH_3$

c) H-Met0$_2$-Glu-His-Phe-D-Lys-Phe-O-$(CH_2)_{10}$-$CH_3$

d) H-Met0$_2$-Glu-His-Phe-D-Lys-Phe-O-$(CH_2)_{11}$-$CH_3$

e) H-Met0$_2$-Glu-His-Phe-D-Lys-Phe-O-$(CH_2)_{12}$-

$CH_3$
f) H-Met$0_2$-Glu-His-Phe-D-Lys-Phe-O-$(CH_2)_{13}$-$CH_3$
g) H-Met$0_2$-Glu-His-Phe-D-Lys-Phe-O-$(CH_2)_{14}$-$CH_3$
h) H-Met$0_2$-Glu-His-Phe-D-Lys-Phe-O-$(CH_2)_{15}$-$CH_3$
i) H-Met$0_2$-Glu-His-Phe-D-Lys-Phe-O-$(CH_2)_{16}$-$CH_3$
j) H-Met$0_2$-Glu-His-Phe-D-Lys-Phe-O-$(CH_2)_{17}$-$CH_3$

EXAMPLE III

In a similar manner (e.g. substituting the appropriate amine for decanol in step 1.1 of EXAMPLE I) are made:

a) H-Met$0_2$-Glu-His-Phe-D-Lys-Phe-NH-$(CH_2)_7$-$CH_3$
b) H-Met$0_2$-Glu-His-Phe-D-Lys-Phe-NH-$(CH_2)_8$-$CH_3$
c) H-Met$0_2$-Glu-His-Phe-D-Lys-Phe-NH-$(CH_2)_9$-$CH_3$
d) H-Met$0_2$-Glu-His-Phe-D-Lys-Phe-NH-$(CH_2)_{10}$-$CH_3$
e) H-Met$0_2$-Glu-His-Phe-D-Lys-Phe-NH-$(CH_2)_{11}$-$CH_3$
f) H-Met$0_2$-Glu-His-Phe-D-Lys-Phe-NH-$(CH_2)_{12}$-$CH_3$
g) H-Met$0_2$-Glu-His-Phe-D-Lys-Phe-NH-$(CH_2)_{13}$-$CH_3$
h) H-Met$0_2$-Glu-His-Phe-D-Lys-Phe-NH-$(CH_2)_{14}$-$CH_3$
i) H-Met$0_2$-Glu-His-Phe-D-Lys-Phe-NH-$(CH_2)_{15}$-$CH_3$
j) H-Met$0_2$-Glu-His-Phe-D-Lys-Phe-NH-$(CH_2)_{16}$-$CH_3$
k) H-Met$0_2$-Glu-His-Phe-D-Lys-Phe-NH-$(CH_2)_{17}$-$CH_3$

## Claims

**1.** A use of a compound having the formula: H-Met$O_2$-Glu-His-Phe-D-Lys-Phe-X-$(CH_2)_nCH_3$, wherein X is NH or O, and n is a positive integer ranging from 7 to 17,
for the manufacture of a medicament for selectively treating central nerve damage.

**2.** The use of claim 1 wherein said medicament is a dosage unit containing at least 150 nanograms of the compound H-Met$O_2$-Glu-His-Phe-D-Lys-Phe-$OC_{10}H_{21}$.

**3.** The use of claim 1 wherein said medicament is an oral dosage unit containing from 50 to 700 micrograms of the compound H-Met$O_2$-Glu-His-Phe-D-Lys-Phe-$OC_{10}H_{21}$.

**4.** The use of claim 2 wherein said compound is incorporated into an implantable device for sustained release of the compound, optionally with other medicaments.

**5.** A use of H-Met$O_2$-Glu-His-Phe-D-Lys-Phe-$OC_{10}H_{21}$ for the manufacture of a medicament for use as an adjuvant for the treatment of central nerve damage.

**6.** A pharmaceutical dosage preparation for the treatment of damage to an eye of an animal, said preparation comprising a compound having the formula:
H-Met$O_2$-Glu-His-Phe-D-Lys-Phe-X-$(CH_2)_nCH_3$, wherein X is NH or O, and n is a positive integer ranging from 7 to 17.

**7.** The pharmaceutical preparation of claim 6 wherein said compound is H-Met$O_2$-Glu-His-Phe-D-Lys-Phe-$OC_{10}H_{21}$ to be administered in a dose of at least 150 nanograms.

**8.** A use of a compound having the formula: H-Met$O_2$-Glu-His-Phe-D-Lys-Phe-X-$(CH_2)_nCH_3$, wherein X is NH or O, and n is a positive integer ranging from 7 to 17,
for the manufacture of a medicament useful in the differential diagnosis of a central versus a peripheral nervous disorder.

**9.** The use of claim 8 wherein said compound is H-Met$O_2$-Glu-His-Phe-D-Lys-Phe-$OC_{10}H_{21}$.

**10.** Each and every novel feature or novel combination of features herein disclosed.

Fig 1

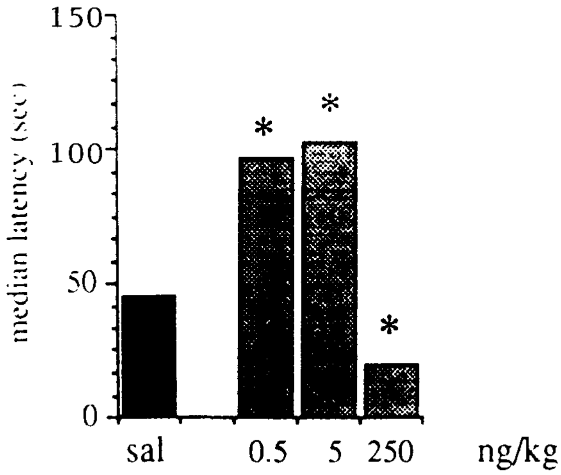
150
100
50
0
median latency (sec)
*
*
*
sal
0.5
5
250
ng/kg

Fig 2

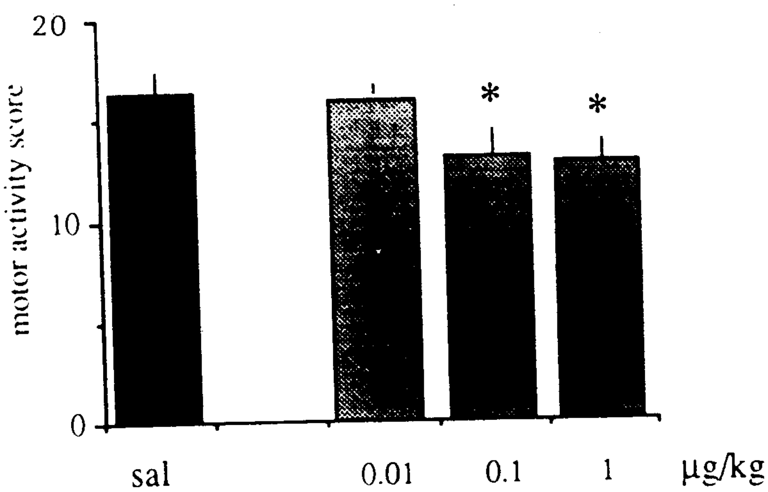
20
10
0
motor activity score
*
*
sal
0.01
0.1
1
µg/kg

Fig 3

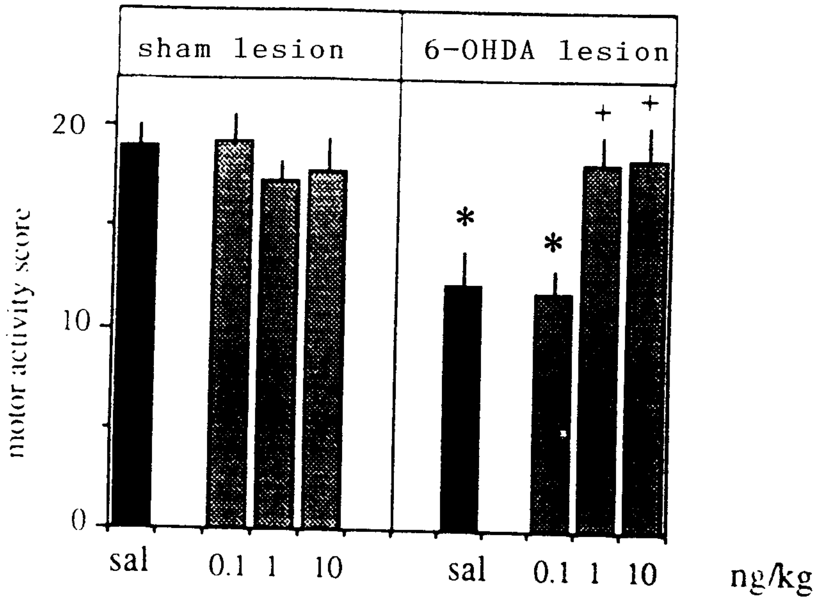

sham lesion
6-OHDA lesion
motor activity score
20
10
0
*
*
+
+
sal
0.1
1
10
sal
0.1
1
10
ng/kg


Fig 4

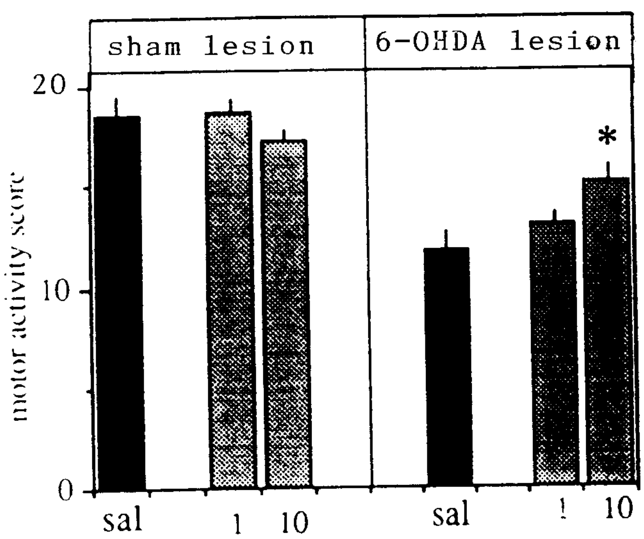

sham lesion
6-OHDA lesion
motor activity score
20
10
0
*
sal
1
10
sal
1
10

European Patent Office

# PARTIAL EUROPEAN SEARCH REPORT

which under Rule 45 of the European Patent Convention shall be considered, for the purposes of subsequent proceedings, as the European search report

Application number
EP 91201552
- page 1 -

## DOCUMENTS CONSIDERED TO BE RELEVANT

| Category | Citation of document with indication, where appropriate, of relevant passages | Relevant to claim | CLASSIFICATION OF THE APPLICATION (Int. Cl 5) |
|---|---|---|---|
| T | BRAIN RESEARCH vol. 527, no. 2, 1990, pages 192-197; B. SPRUIJT et al.: "Org2766 improves performance of rats with unilateral lesions in the fimbria fornix in a spatial learning task" * abstract * | 1-9 | A61K37/40<br>C07K15/00<br>C07K7/06 |
| A | WO-A-8203388 (AKZO) * the whole document *; & US-A-4550099 (cat. D) | 1-9 | |
| A,D | US-A-3842064 (H.M GREVEN) * column 5 * | 1-9 | |
| A,D | US-A-4110322 (H.M. GREVEN et al.) * the whole document * | 1-9 | |
| | ... | | |

TECHNICAL FIELDS SEARCHED (Int. Cl 5)

A61K
C07K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-9
Claims searched incompletely: –
Claims not searched: 10
Reason for the limitation of the search:

Article 84 - EPC -

| Place of search | Date of completion of the search | Examiner |
|---|---|---|
| Berlin | 26.07.1991 | P. AVEDIKIAN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number
EP 91201552
- page 2 -

## DOCUMENTS CONSIDERED TO BE RELEVANT

| Category | Citation of document with indication, where appropriate, of relevant passages | Relevant to claim | CLASSIFICATION OF THE APPLICATION (Int. Cl.) |
|---|---|---|---|
| A,D | US-A-4104371 (H.M. GREVEN et al.)<br>* the whole document * | 1-9 | |
| A | EP-A-0179332 (HOECHST)<br>* the whole document * | 1-9 | |
| A | NEUROENDOCRINOLOGY LETTERS<br>vol. 10, no. 4, 1988, page 237;<br>M. DIAMANT et al.: "In vitro stereoido-genic and trophic activity of ACTH and ACTH-related peptides"<br>* the whole document * | 1-9 | |

TECHNICAL FIELDS SEARCHED (Int. Cl.)